# EUROPEAN PATENT APPLICATION

(11) **EP 3 841 998 A1**
(43) Date of publication of application: **30.06.2021**
(21) Application number: 20216536.1
(22) Date of filing: 22.12.2020
(51) Int. Cl.: A61B 18/14, A61B 18/00

(54) **LASSO CATHETER WITH BALLOON**

(30) Priority: 23.12.2019 US 201916726110
(71) Applicant: Biosense Webster (Israel) Ltd, Yokneam, 2066717 (IL)
(72) Inventor: GOVARI, Assaf, Yokneam 2066717 (IL)
(74) Representative: Small, Gary James

(57) **Abstract**

Embodiments of the present invention include a medical apparatus having an insertion tube, a flexible probe, a plurality of electrodes and a balloon. The insertion tube includes a distal end configured for insertion into a body cavity and containing a lumen passing through the insertion tube. The flexible probed is configured to be deployed from the distal end of the insertion tube and to assume an arcuate shape upon deployment within the body cavity. The electrodes are distributed along the probe, and the balloon is configured to have a portion of the balloon surrounded by the arcuate-shaped probe and to be inflated by passage of a fluid through the lumen while the probe is deployed in the body cavity.

## Description

### FIELD OF THE INVENTION

The present invention relates generally to invasive catheters, and specifically to an invasive medical catheter comprising a lasso-shaped distal and a balloon that can be inflated to increase the diameter of the lasso-shaped distal end.

### BACKGROUND OF THE INVENTION

Ablation of myocardial tissue is well known as a treatment for cardiac arrhythmias. In radio-frequency (RF) ablation, for example, a catheter is inserted into the heart and brought into contact with tissue at a target location. RF energy is then applied through an electrode on the catheter in order to create a lesion for the purpose of breaking arrhythmogenic current paths in the tissue. In some ablation procedures used to treat atrial arrhythmias (particularly for atrial fibrillation), a lasso catheter can be used to perform circumferential ablation of the ostia of the pulmonary veins.

U.S. Patent 9,265,575 to Coe et al., describes a balloon catheter neuromodulation system. The balloon catheter includes a bipolar electrode pair, wherein at least one of the bipolar electrode pair is configured to be positioned to be expanded into contact with an inner wall of the hepatic artery branch upon expansion of the at least one expandable balloon.

U.S. Patent Application 2016/0235477 to Shutaro describes a balloon catheter ablation system. The system includes a highly directional pressure sensor that is provided coaxially in an anterior portion of a catheter shaft within a balloon. This enables monitoring a pressing force against the balloon onto the tissue, a temperature of the balloon, impedances, potentials, and an energization time. Additionally, providing the directional pressure sensor inside the balloon enables a pressing force from the balloon onto the tissue to be monitored with accuracy without being influenced by the swirl's liquid, thereby securing effective ablation of the target tissues.

U.S. Patent Application 2015/0141982 to Lee describes a multi-electrode balloon catheter with circumferential and point electrodes. The balloon catheter includes outer and inner balloon member, which can be elastic or inelastic. This allows the members to inflate and expand outwardly under an internal force and to deflate and collapse when the force is absent or removed. The internal force is provided by introduction of an inflation medium into a cavity of the inner balloon member.

U.S. Patent 5,984,917 to Fleischman et al., describes a device and method for remote insertion of a closed loop of a lasso-shaped catheter. The device includes a catheter mechanism used to expand a metallic mesh during insertion over an inverted appendage.

U.S. Patent 5,984,917 to Grunewald describes a catheter with multiple electrode assemblies for use at or near tubular regions of the heart. The catheter includes a distal electrode assembly and a proximal electrode assembly. The distal electrode assembly has an elongated member defining a longitudinal axis and a plurality of spines surrounding the member and converging at their proximal and distal ends, where each spine has at least one electrode and a curvature so that the spine bows radially outwardly from the member. The proximal electrode assembly has a proximal electrode assembly has an elongated member configured with a generally radial portion and a generally circular portion generally transverse to the catheter axis, where the generally circular portion comprising a plurality of electrodes.

U.S. Patent 5,984,917 to Fleischman et al., describes a device and method for remote insertion of a lasso portion (i.e., assembly) of a catheter. The catheter includes a flexible catheter body having a distal tip portion. A lumen or tubular guide is provided for allowing the lasso to be freely axially movable so that the lasso can be expanded or contracted.

U.S. Patent 5,984,917 to Ditter et al., describes a catheter with a variable arcuate distal section. The catheter includes a mandrel contained within a hollow support member, the distal end has a generally circular form, and an operator can expand or even significantly straighten the form of the distal assembly by advancing the mandrel through the hollow support member.

The description above is presented as a general overview of related art in this field and should not be construed as an admission that any of the information it contains constitutes prior art against the present patent application.

### SUMMARY OF THE INVENTION

There is provided, in accordance with an embodiment of the present invention, a medical apparatus, including an insertion tube having a distal end configured for insertion into a body cavity and containing a lumen passing through the insertion tube, a flexible probe configured to be deployed from the distal end of the insertion tube and to assume an arcuate shape upon deployment within the body cavity, a plurality of electrodes distributed along the probe, and a balloon configured to have a portion of the balloon surrounded by the arcuate-shaped probe and to be inflated by passage of a fluid through the lumen while the probe is deployed in the body cavity.

In some embodiments, the balloon, when inflated, exerts an outward force against the arcuate-shaped probe so as to press the electrodes against tissue in the body cavity.

In further embodiments, the body cavity includes a pulmonary vein, and the tissue includes intravenous tissue. In one embodiment, inflating the balloon forms a seal between the balloon and the intravenous tissue so as to prevent blood flowing through the pulmonary vein from coming in contact with the electrodes.

In additional embodiments, a given electrode is configured to convey ablation energy to tissue in the body cavity in contact with the given electrode.

In supplementary embodiments, a given electrode includes perforations configured to deliver an irrigation fluid to the tissue.

In other embodiments, a given electrode is configured to generate a signal indicating an electrical potential in tissue in the body cavity in contact with the electrode.

In supplementary embodiments, the medical apparatus may also include an extender shaft contained within the insertion tube, affixed to a distal end of the balloon, and configured to position the balloon within the arcuate-shaped probe when extended from the insertion tube. In one embodiment, the medical apparatus may additionally include a position transducer affixed to the extender shaft.

In some embodiments, the arcuate shape has a radius of curvature between 15mm and 30mm.

There is also provided, in accordance with an embodiment of the present invention, a method for fabricating a catheter including providing an insertion tube having a distal end configured for insertion into a body cavity and containing a lumen passing through the insertion tube, providing a flexible probe configured to be deployed from the distal end of the insertion tube and to assume an arcuate shape upon deployment within the body cavity, distributing a plurality of electrodes along the probe and providing a balloon configured to have a portion of the balloon surrounded by the arcuate-shaped probe and to be inflated by passage of a fluid through the lumen while the probe is deployed in the body cavity.

There is additionally provided, in accordance with an embodiment of the present invention, method for treatment, including inserting, into a body cavity, an insertion tube having a distal end containing a lumen passing through the insertion tube, deploying, into the body cavity from the distal end, an arcuate shaped flexible probe including a plurality of electrodes, deploying, from the insertion tube, a balloon to within the arcuate-shaped probe, inflating, by passing a fluid through the lumen, the balloon, thereby exerting an outward force against the arcuate-shaped probe so as press the electrodes against tissue in the body cavity, and performing, using the electrodes, a medical procedure on the tissue.

In one embodiment, inflating the balloon exerts an outward force against the arcuate-shaped probe so as to press the electrodes against tissue in the body cavity.

### BRIEF DESCRIPTION OF THE DRAWINGS

The disclosure is herein described, by way of example only, with reference to the accompanying drawings, wherein:
Figure 1 is a schematic, pictorial illustration of a medical system comprising a catheter and a control console, in accordance with an embodiment of the present invention;
Figure 2 is a schematic cutaway view of a distal end of the catheter comprising a medical probe having an arcuate-shaped end section and a balloon that can be deployed within the arcuate-shaped end section, in accordance with an embodiment of the present invention;
Figure 3 is a flow diagram that schematically illustrates a method of performing a medical procedure using the arcuate-shaped end section and the balloon, in accordance with an embodiment of the present invention;
Figure 4 is a schematic pictorial illustration of the distal end of the catheter positioned in a pulmonary vein, in accordance with an embodiment of the present invention;
Figure 5 is a schematic pictorial illustration of the arcuate-shaped end section of the medical probe deployed within the pulmonary vein, in accordance with an embodiment of the present invention; and
Figure 6 is a schematic pictorial illustration of the balloon deployed within the arcuate-shaped end section, in accordance with an embodiment of the present invention.

### DETAILED DESCRIPTION OF EMBODIMENTS

Ablation and mapping are examples of medical procedures that can be performed on a pulmonary vein by electrodes distributed on a lasso catheter. However, even when the lasso catheter is positioned correctly within the pulmonary vein, all the electrodes of lasso catheter may not contact the tissue of the vein. Additionally, even if there is contact, the contact may not be good enough for ablation and/or for good signal acquisition (i.e., for mapping).

Embodiments of the present invention provide a medical apparatus that combines an arcuate-shaped (i.e., a lasso-shaped) end section with a balloon that can be used to expand the diameter of the arcuate-shaped end section. As described hereinbelow, the medical apparatus comprises an insertion tube, a flexible probe, a plurality of electrodes distributed along the probe, and a balloon. The flexible probe has a distal end configured for insertion into a body cavity and contains a lumen passing through the insertion tube, and the flexible probe is configured to assume an arcuate shape upon being deployed from the distal end of the insertion tube into the body cavity. The balloon is configured to have a portion of the balloon surrounded by the arcuate-shaped probe, and to be inflated by passage of a fluid through the lumen while the probe is deployed in the body cavity.

In some embodiments inflating the balloon exerts an outward force against the arcuate-shaped probe so as press the electrodes against tissue in the body cavity. Therefore, medical systems implementing embodiments of the present invention can reduce, or even completely prevent, contact of the parts of the electrodes that are not facing the vein with the blood pool in the vein. This is advantageous when using the electrodes for medical procedures comprising ablation and/or signal acquisition.

### SYSTEM DESCRIPTION

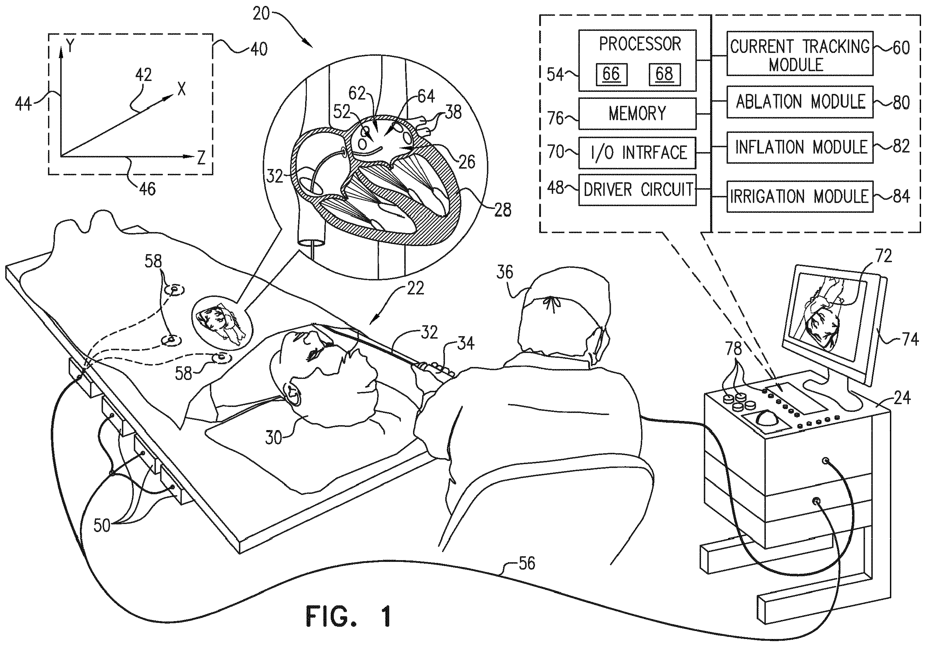

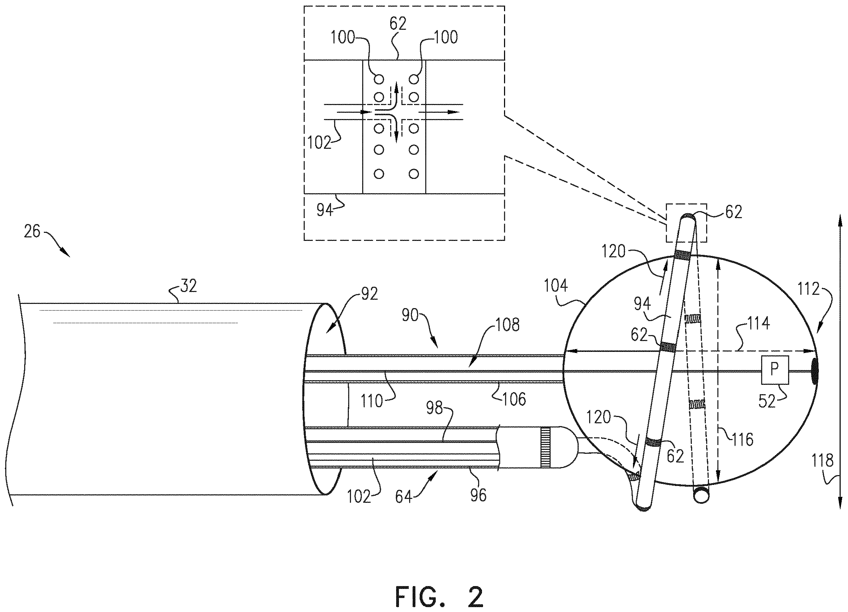

Figure 1 is a schematic, pictorial illustration of a medical system 20 comprising a catheter 22 and a control console 24, in accordance with an embodiment of the present invention. As described in more detail in Figure 2 hereinbelow, catheter 22 comprises a flexible medical probe that assumes an arcuate shape upon deployment from a distal end 26 of the catheter, and a balloon that can be deployed from the distal end and positioned within the arcuate-shaped probe. Medical system 20 may be based, for example, on the CARTO® system, produced by Biosense Webster Inc. of 33 Technology Drive, Irvine, California, U.S.A.

In embodiments described hereinbelow, catheter 22 can be used for diagnostic or therapeutic treatment. In one embodiment, medical system 20 can use catheter 22 for mapping electrical potentials of a heart 28 of a patient 30. In another embodiment, medical system 20 can use catheter 22 for ablation of tissue in heart 28. Alternatively, catheter 22 may be used, *mutatis mutandis,* for other therapeutic and/or diagnostic purposes in the heart or in other body organs.

Catheter 22 comprises an insertion tube 32 and a handle 34 coupled to a proximal end of the insertion tube. By manipulating handle 34, a medical professional 36 can insert catheter 22 into a body cavity in patient 30. For example, medical professional 36 can insert catheter 22 through the vascular system of patient 30 so that distal end 26 of catheter 22 enters a chamber of heart 28 or a given pulmonary vein 38 and engages myocardial or intravenous tissue at a desired location or locations.

In the configuration shown in Figure 1, system 20 uses magnetic-based position sensing and/or impedance-based location sensing. System 20 can use magnetic-based position sensing to determine position coordinates indicating a location and an orientation of distal end 26 in a coordinate system 40 comprising an X-axis 42, a Y-axis 44 and a Z-axis 46, and the medical system can use impedance-based location sensing to determine location coordinates of the distal end in the coordinate system.

To implement magnetic based position sensing, control console 24 comprises a driver circuit 48 which drives field generators 50 to generate magnetic fields within the body of patient 30. Typically, field generators 50 comprise coils, which are placed below the patient's torso at known positions external to patient 30. These coils generate magnetic fields in a predefined working volume that contains heart 28.

Medical system 20 also comprises a position transducer such as a magnetic field sensor 52 that is associated with catheter 22 and a processor 54 in medical console 24. The association is described in more detail below, with reference to Figure 2. In response to the magnetic fields from the field generator coils, magnetic field sensor 52 generates and conveys electrical signals indicating a current position (i.e., location and orientation) of distal end 26, and processor 54 is configured to receive and process the conveyed signals in order to compute, in coordinate system 40, orientation and location coordinates of the distal end.

Magnetic position tracking techniques are described, for example, in U.S. Patents 5,391,199, 5,443,489, 6,788,967, 5,558,091, 6,172,499 and 6,177,792. The methods of location sensing described hereinabove are implemented in the above-mentioned CARTO® system and are described in detail in the patents cited above.

As described supra, medical system 20 can also use impedance-based location sensing to determine location coordinates of distal end 26 in coordinate system 40. Control console 24 is connected, by a cable 56, to body surface electrodes, which typically comprise adhesive skin patches 58 that are affixed to patient 30. In the configuration shown in Figure 1, cable 56 also connects field generators 50 to console 24. Control console 24 also comprises a current tracking module 60 that, in conjunction with processor 54, determines position coordinates of distal end 26 inside heart 28 based on impedances measured between adhesive skin patches 58 and electrodes 62 that are affixed to a medical probe 64, as shown in Figure 2.

Impedance-based position tracking techniques are described, for example, in U.S. Patents 5,983,126, 6,456,864 and 5,944,022. The methods of position sensing described hereinabove are implemented in the above-mentioned CARTO® system and are described in detail in the patents cited above.

In embodiments described herein, electrodes 62 can also be configured to apply a signal to tissue in heart 28 or a given pulmonary vein 38, and/or to measure a certain physiological property (e.g., the local surface electrical potential) at a location in the heart or the given pulmonary vein. In additional embodiments, electrodes 62 can be configured to deliver ablation energy to the tissue in heart 28 or a given pulmonary vein 38. Electrodes 62 are connected to control console 24 by wires (not shown) running through probe 64.

Processor 54 may comprise real-time noise reduction circuitry 66 typically configured as a field programmable gate array (FPGA), followed by an analog-to-digital (A/D) ECG (electrocardiograph) signal conversion integrated circuit 68. The processor can pass the signal from A/D ECG circuit 68 to another processor and/or can be programmed to perform one or more algorithms disclosed herein, each of the one or more algorithms comprising steps described hereinbelow. The processor uses circuitry 66 and circuit 68, as well as features of modules which are described in more detail below, in order to perform the one or more algorithms.

Control console 24 also comprises an input/output (I/O) communications interface 70 that enables the control console to transfer signals from, and/or transfer signals to magnetic field sensor 52, electrodes 62 and adhesive skin patches 58. Based on signals received from magnetic field sensor 52 and/or electrodes 62 and/or adhesive skin patches 58, processor 54 can generate a map 72 that shows the position of distal end 26 in the patient's body.

During a procedure, processor 54 can present map 72 to medical professional 36 on a display 74, and store data representing the map in a memory 76. Memory 76 may comprise any suitable volatile and/or non-volatile memory, such as random-access memory or a hard disk drive.

In some embodiments, medical professional 36 can manipulate map 72 using one or more input devices 78. In alternative embodiments, display 74 may comprise a touchscreen that can be configured to accept inputs from medical professional 36, in addition to presenting map 72.

In the configuration shown in Figure 1, control console 24 also comprises an ablation module 80, an inflation module 82 and an irrigation module 84, whose respective functionalities are described in the description referencing Figure 2 hereinbelow.

Figure 2 is a schematic cutaway view of distal end 26 of catheter 22, in accordance with an embodiment of the present invention. In the configuration shown in Figure 2, catheter 22 comprises medical probe 64, also termed herein a lasso probe or a lasso catheter, and a balloon probe 90. Medical probe 64 and balloon probe 60 are contained within insertion tube 32 and are configured to extend from a lumen 92 in the insertion tube at distal end 26.

Medical probe 64 comprises an end section 94 that is affixed to a tubular shaft 96. End section 94 is configured to form an arcuate shape when deployed from lumen 92 and comprises a plurality of cylinder-shaped electrodes 62 disposed along its length. Electrodes 62 are connected to console 24 by wires 98 running through tubular shaft 96.

In one embodiment, as described supra, medical system 20 can use electrodes 62 and adhesive skin patches 58 to determine location coordinates of end section 94 in patient 30. In another embodiment, electrodes 62 can be configured to convey (i.e., to processor 54) signals indicating electrical potentials in tissue in contact with the electrodes.

In a further embodiment as described supra, medical system 20 can use electrodes 62 to ablate tissue in heart 28 and/or a given pulmonary vein 38. In these embodiments, ablation module 80 can generate and control delivery of ablation energy (e.g., radio-frequency energy) to electrodes 62 via I/O interface 70.

In embodiments where medical system 20 uses electrodes 62 for tissue ablation, the electrodes may have multiple perforations 100 through which irrigation fluid (e.g., a saline solution) may be delivered to the tissue with which the electrodes are in contact during ablation. The irrigation fluid can be delivered via an irrigation lumen 102 that is contained within tubular shaft 96 and connected to irrigation module 84. Irrigation module 84 is configured to force the irrigation fluid into irrigation lumen 102 at a controllable pumping rate.

The arcuate shape of end section 94 may be maintained, for example, by incorporating a thin strut made from a shape memory material, such as Nitinol (not shown in the figures), in the desired shape within the end section. The strut is made sufficiently flexible to permit the end section to straighten during insertion and withdrawal through insertion tube 32, but to resume its arcuate form when it is unconstrained inside a body cavity of patient 30. The radius of curvature of end section 94, when unconstrained (i.e., when deployed from lumen 92), is typically between 15 mm and 30 mm.

Balloon probe 90 comprises a balloon 104 that is affixed to a tubular shaft 106. Balloon 104 is typically formed from bio-compatible material such as polyethylene terephthalate (PET), polyurethane, Nylon, or Pebax.

In some embodiments, inflation module 82 can pump, via an inflation lumen 108 contained in shaft 106, a fluid (e.g., normal saline) into balloon 104 so as to inflate the balloon.

In the configuration shown in Figure 2, balloon probe 90 comprises an extender shaft 110 and magnetic field sensor 52 is affixed to the extender shaft. Extender shaft 110 is contained within tubular shaft 106 and is coupled to a distal end 112 of balloon 104. In operation, medical professional 36 can control a length 114 of balloon 104 (i.e., once the balloon is deployed from lumen) by extending or extracting extender shaft 110, and the medical professional can control a width 116 of the balloon by specifying, to inflation module 82, a volume of irrigation fluid to deliver into the balloon.

While the configuration in Figure 2 shows the position transducer comprising magnetic field sensor 52, other types of position transducers are considered to be within the spirit and scope of the present invention. For example, the position transducer may comprise a pair of additional electrodes that are distributed along extender shaft 110, which together can be used by current tracking module 60 and processor 54, typically after a calibration process, to determine location and orientation of distal end 112.

In operation, extender shaft 110 is configured (i.e., when extended from insertion tube 32) to deploy balloon 104 from shaft 106, and to position the balloon such that portions of the balloon are surrounded by arcuate-shaped end section 94. Additionally, in embodiments of the present invention, medical professional 36 can control a diameter of arcuate-shaped end section 94 by controlling the pressure of the fluid used to inflate the balloon. Inflating balloon 104 increases width 116, thereby exerting an outward force 120 against arcuate-shaped end section 94 so as to increase the diameter of the arcuate-shaped end section.

As described hereinabove, end section 94 of medical probe 64 is configured to be deployed from lumen 92, extender shaft 106 is configured to be deployed from tubular shaft 106, and balloon 104 is configured to be inflated by fluid delivered via inflation lumen 108. In one embodiment, medical professional 36 can use input devices 78 to manage one or more of these operations. In another embodiment, handle 34 may comprise one or more controls (not shown) to manage one or more of these operations.

### LASSO CATHETER DEPLOYMENT AND SIZING

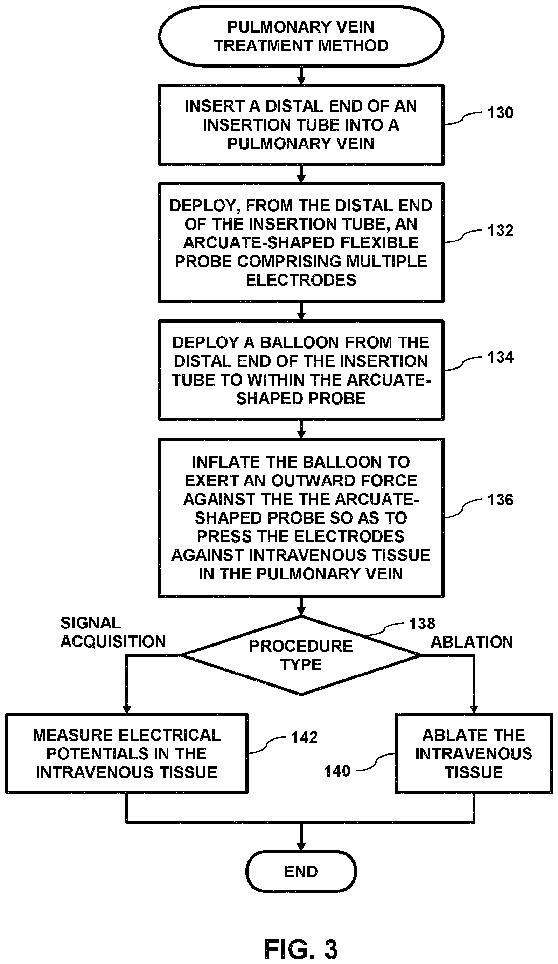

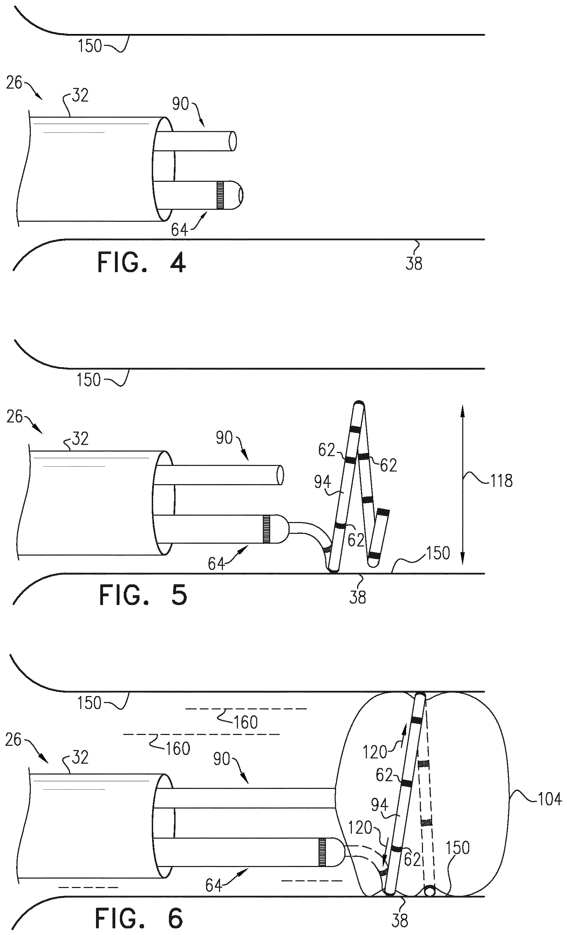

Figure 3 is a flow diagram that schematically illustrates a method using catheter 22 to perform a medical procedure in heart 28, and Figure 4 is a schematic pictorial illustration of distal end 26 in a given pulmonary vein 38, in accordance with an embodiment of the present invention.

In an insertion step 130, medical professional 36 manipulates, using handle 34, catheter 22 so as to insert distal end 26 into the given pulmonary vein. As shown in Figure 4, when initially inserting distal end 26 into the given pulmonary vein, probes 64 and 90 are typically still recessed (i.e., not deployed) within insertion tube 32.

In a first deployment step 132, medical professional 36 deploys, from the distal end of insertion tube 32, end section 94 of medical probe 64 into the given pulmonary vein. As described supra, upon deploying end section 94 from the distal end of insertion tube 32, the end section assumes an arcuate shape.

Figure 5 is a schematic pictorial illustration showing arcuate-shaped end section 94 deployed into the given pulmonary vein. As shown in Figure 5, upon assuming an arcuate shape, diameter 118 of end section 94 may not be sufficient to enable all electrodes 62 to engage intravenous tissue 150.

In a second deployment step 134, medical professional 36 deploys balloon 104, in its uninflated state from the distal end of insertion tube 32 to within arcuate-shaped end section 94. The balloon is deployed by extending extender shaft 110 distally, and processor 58 checks that the balloon is within section 54 using position measurements from sensor 52, and positions of electrodes 62. In some embodiments processor 58 may provide a notification to professional 36, by any convenient means such as a notice on display 74, of correct deployment of balloon 104.

In an inflation step 136, the medical professional conveys an instruction (e.g., using input devices 78) to inflation module 82 to inflate the balloon. In embodiments of the present invention, inflating balloon 104 exerts outward force 120 on end section 94 so that all electrodes 62 press against (i.e., engage) intravenous tissue 150.

Figure 6 is a schematic pictorial illustration showing arcuate-shaped end section 94 deployed into the given pulmonary vein, and balloon 104 deployed within the arcuate-shaped end section and inflated so that outward force 120 presses electrodes 62 against intravenous tissue 150. As shown in Figure 6, the flexible property of balloon 104 causes the balloon to envelop end section 94 and form a seal between the balloon and intravenous tissue 150 as outward force 120 presses electrodes 62 against the intravenous tissue. Therefore electrodes 62 are in contact with balloon 104 and electrodes 62 but are not in contact with blood 160 flowing through the pulmonary vein. This is advantageous when the electrodes are used for medical procedures such as ablation or signal acquisition, as described hereinbelow.

In a treatment selection step 138, if the medical procedure to be performed is ablation, then in an ablation step 140, medical professional conveys an instruction to ablation module 80 to deliver ablation energy to electrodes 62, which in turn, conveys the ablation energy to the intravenous tissue in contact with the electrodes, and the method ends. Returning to step 138, if the medical procedure to be performed is signal acquisition, then in a mapping step 142, medical professional 36 conveys an instruction to processor 54 to measure electrical potentials at locations on intravenous tissue 150 that are engaged by electrodes 62, and the method ends.

It will be appreciated that the embodiments described above are cited by way of example, and that the present invention is not limited to what has been particularly shown and described hereinabove. Rather, the scope of the present invention includes both combinations and subcombinations of the various features described hereinabove, as well as variations and modifications thereof which would occur to persons skilled in the art upon reading the foregoing description and which are not disclosed in the prior art.

### ASPECTS OF THE INVENTION

1. A method for treatment, comprising:
   inserting, into a body cavity, an insertion tube having a distal end containing a lumen passing through the insertion tube;
   deploying, into the body cavity from the distal end, an arcuate shaped flexible probe comprising a plurality of electrodes;
   deploying, from the insertion tube, a balloon to have a portion of the balloon surrounded by the arcuate-shaped probe;
   inflating, by passing a fluid through the lumen, the balloon, thereby exerting an outward force against the arcuate-shaped probe so as press the electrodes against tissue in the body cavity; and
   performing, using the electrodes, a medical procedure on the tissue.
2. The method according to aspect 1, wherein inflating the balloon exerts an outward force against the arcuate-shaped probe so as to press the electrodes against tissue in the body cavity.

## Claims

1. A medical apparatus, comprising:
an insertion tube having a distal end configured for insertion into a body cavity and containing a lumen passing through the insertion tube;
a flexible probe configured to be deployed from the distal end of the insertion tube and to assume an arcuate shape upon deployment within the body cavity;
a plurality of electrodes distributed along the probe; and
a balloon configured to have a portion of the balloon surrounded by the arcuate-shaped probe and be inflated by passage of a fluid through the lumen while the probe is deployed in the body cavity.

2. The medical apparatus according to claim 1, wherein the balloon, when inflated, exerts an outward force against the arcuate-shaped probe so as to press the electrodes against tissue in the body cavity.

3. The medical apparatus according to claim 1, wherein a given electrode is configured to convey ablation energy to tissue in the body cavity in contact with the given electrode, a given electrode comprises perforations configured to deliver an irrigation fluid to the tissue, or a given electrode is configured to generate a signal indicating an electrical potential in tissue in the body cavity in contact with the electrode.

4. The medical apparatus according to claim 1, and comprising an extender shaft contained within the insertion tube, affixed to a distal end of the balloon, and configured to position the balloon within the arcuate-shaped probe when extended from the insertion tube.

5. The medical apparatus according to claim 4, and comprising a position transducer affixed to the extender shaft.

6. The medical apparatus according to claim 1, wherein the arcuate shape has a radius of curvature between 15mm and 30mm.

7. A method for fabricating a catheter, comprising:
providing an insertion tube having a distal end configured for insertion into a body cavity and containing a lumen passing through the insertion tube;
providing a flexible probe configured to be deployed from the distal end of the insertion tube and to assume an arcuate shape upon deployment within the body cavity;
distributing a plurality of electrodes along the probe; and
providing a balloon configured to have a portion of the balloon surrounded by the arcuate-shaped probe and be inflated by passage of a fluid through the lumen while the probe is deployed in the body cavity.

8. The method according to claim 7, wherein inflating the balloon exerts an outward force against the arcuate-shaped probe so as to press the electrodes against tissue in the body cavity.

9. The medical apparatus according to claim 2 or the method according to claim 8, wherein the body cavity comprises a pulmonary vein, and wherein the tissue comprises intravenous tissue.

10. The medical apparatus or the method according to claim 9, wherein inflating the balloon forms a seal between the balloon and the intravenous tissue so as to prevent blood flowing through the pulmonary vein from coming in contact with the electrodes.

11. The method according to claim 7, and comprising conveying, by a given electrode, ablation energy to tissue in the body cavity in contact with the given electrode, or comprising generating, by a given electrode, a signal indicating an electrical potential in tissue in the body cavity in contact with the electrode.

12. The method according to claim 7, wherein a given electrode comprises perforations, and comprising delivering, via the perforations, an irrigation fluid to the tissue.

13. The method according to claim 7, wherein the catheter comprises an extender shaft contained within the insertion tube and affixed to a distal end of the balloon, and comprising positioning, by extending the extender shaft from the insertion tube, the balloon having a portion surrounded by the arcuate-shaped probe.

14. The method according to claim 13, wherein the catheter comprises a position transducer affixed to the extender shaft.

15. The method according to claim 7, wherein the arcuate shape has a radius of curvature between 15mm and 30mm.
